(19) Europäisches Patentamt
European Patent Office
Office européen des brevets

(11) **EP 3 731 233 A1**

(12) **EUROPEAN PATENT APPLICATION**

(43) Date of publication:
**28.10.2020 Bulletin 2020/44**

(51) Int Cl.:
**G16H 20/17** *(2018.01)*      **G16H 50/20** *(2018.01)*

(21) Application number: **19170910.4**

(22) Date of filing: **24.04.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Digital Diabetes Analytics Sweden AB 723 49 Västerås (SE)**

(72) Inventor: **CEDERBLAD, Lars 723 49 VÄSTERÅS (SE)**

(74) Representative: **Bjerkéns Patentbyrå KB (Stockholm)
Box 5366
102 49 Stockholm (SE)**

(54) **DECISION SUPPORT SYSTEM, AND METHOD IN RELATION THERETO**

(57)      A decision support system configured to support patients with Type 1 Diabetes, T1D, in their daily decision making, to determine a decision advice including timing and dosage of insulin, the support system comprises a primary module (2) facilitating a primary loop related to each patient belonging to a community of several patients with T1D, the primary module (2) comprises a primary feature estimation block (4), a feature augmentation block (6), a decision advice block (8), and a control unit (10).

The system further comprises a secondary module (12) facilitating a secondary loop being a learning loop configured to apply organized data from said community of several patients with T1D, and that said primary module (2) and said secondary module (12) are involved in the decision making for each patient belonging to said community, wherein said secondary module (12) comprises a clustering of patients block (14), and a feature estimation per cluster block (16).

FIG. 2

**Description**

Technical field

The present disclosure relates to a patient-centered decision support system for health and medical application based on double-loop learning, and in particular a system for supporting patients with Type 1 Diabetes (T1D) in their daily decision-making that augments the patient-specific estimates with estimates from data and features from a cluster of similar patients.

Background

Due to the chronic nature of T1D, it is a lifelong challenge that requires the patient to continuously reflect and act to keep a proper balance of blood glucose (BG) levels. The risk for hypoglychemia or late complications is an additional challenge that is an incentive, a threat and motivation factor for the right balance.

The balance of BG around the clock depends on a large variety of factors, where the standard factors such as food and insulin are only the base. The list of factors contains parameters as listed in the following: Food intake (mainly carbohydrates (carbs)), bolus insulin, basal insulin, actual BG level, exercise, stress, weight, alcohol, menstrual period, environmental temperature, and other factors not yet understood.

The advice given by doctors can be rather basic, i.e., "use 4-6 U (Units) for a standard breakfast", based on experience with many patients. Some standard rules exist:

1) How to calculate the amount of insulin needed for a certain amount of carbs.
2) How to correct a high BG.

However, these rules are basic and static.

In the following some prior art documents disclosing various solutions in this technical field will be briefly discussed.

US20130345663 describes a decision-making management system for recommending an appropriate bolus dosage. The document further discusses the advantages of using data from multiple patients to create a group pattern when treating a particular patient having a similar pattern as the group.

US20160070871 discloses a method for recommending a bolus dosage to a patient. A processing unit together with an analytic unit are configured to determine the optimal dosage based on a plurality of bolus dosages obtained from a plurality of bolus calculators. US20120123234 discloses a monitoring method/system for decision making for diabetes treatment. The method obtains reference data including individualized patient's profile and patient's history data. The system applies a self-learning approach for up-dating the patient's profile. Data is collected e.g. during several days prior to connecting the patient to the monitoring system.

US20050278073 and WO201811766 show learning methods in general for estimating when the patient should take the bolus based on time patterns and reinforcement learning. US2017216518 relates to system and methods relating to open loop decision-making using e.g. cloud or big data, for management of diabetes. As an example of the use of cloud or big data, a health care professional (HCP, e.g., doctor, physician, nurse, caregiver) may desire to determine initial values for insulin resistance or insulin sensitivity. The HCP may enter demographic data such as weight, age, and gender. An application program, an app, may then search through a database and find users most similar to the subject user, and may take an average of their settings for initial suggestions. Cloud or big data may further be employed for initial risk stratification, again based on aggregate numbers.

One commonly used sample device used by patients with diabetes to monitor glucose is a self-monitoring blood glucose (SMBG) monitor, which typically uses finger pricks to obtain blood samples for measurement. Another device used to monitor glucose is a continuous analyte sensor, which typically includes a sensor that is placed subcutaneously, transdermally (e.g., transcutaneously), or intravascularly. The sensor measures the concentration of a given analyte within the body, and generates a raw signal that is transmitted to electronics associated with the sensor. The raw signal is converted into an output value that is displayed. The output value that results from the conversion of the raw signal is typically expressed in a form that provides the user with meaningful clinical information, such as glucose expressed in mmol/l.

The present disclosure relates to systems and methods for decision-making for management of diabetes. People with diabetes face many problems in controlling their glucose because of the complex interactions between food, insulin, exercise, stress, activity, and other physiological and environmental conditions. Established principles of management of glucose sometimes are not adequate because there is a significant amount of variability in how different conditions impact different individuals and what actions might be effective for them.

The systems and methods according to present disclosure may be applied to any type of analyte monitoring device, e.g., SMBGs, Flash Glucose Meters (FGMs) and Continuous Glucose Meters (CGMs), or other types of sensor systems including those with invasive sensors, noninvasive sensors, and implantable sensors. A glucose sensor may measure a concentration of glucose or a substance indicative of the concentration or presence of another analyte. The sensor may be a continuous device, for example a subcutaneous, transdermal, transcutaneous, non-invasive, intraocular and/or intravascular (e.g., intravenous) device. A glucose sensor can use any method of glucose measurement, including enzymatic, chemical, physical, electrochemical, optical, optochemical, fluorescence-based, spectrophotometric, spectroscopic (e.g., optical absorption spectroscopy, Raman spectroscopy, etc.), polarimetric, calorimetric, iontophoretic, radiometric, and the like. The glucose sensor can use any known detection method, including invasive, minimally invasive, and non-invasive sensing techniques, to provide a data stream indicative of the concentration of the analyte in a host. The data stream is typically a raw data signal that is used to provide a useful value of the analyte to a user, such as a patient or health care professional (HCP, e.g., doctor, physician, nurse, caregiver), who may be using the sensor.

The object of the present invention is to achieve an improved system and method to provide an improved and accurate decision advice for a patient having T1D to keep a proper balance of blood glucose level all the time, in order to lower the risk for both hypoglycaemia and long-term complications.

Summary

The above-mentioned object is achieved, or at least alleviated, by the present invention according to the independent claims.

Preferred embodiments are set forth in the dependent claims.

The present disclosure describes a way of supporting people with T1D in their daily decision making. Management of stable blood glucose is a delicate balance that must be managed by the patient rather than the health care provider. The balance involves several trade-offs; e.g., tight blood glucose control vs. long-term complications, freedom of life vs. a uniform and sometimes boring way of living.

Thus, the object of the present invention is achieved by providing the patient with a personal decision support system that is augmented with learnings from many patients with similar physiological and living conditions. The concept may also include the identification of typical situations where a clinical specialist may identify corrective actions. These corrective actions are given as feedback to the patients in parallel to the decisions support.

The suggested concept comprises interaction between a primary and a secondary loop. An improved support system would then be achieved where the improvement lies in that the individual settings are tailored as described in the suggested concept, i.e. how the interaction between the loops is performed.

The concept presented is based on a double loop learning system that will help the patient to take the right decisions with regards to carbs (carbohydrates) intake vs. insulin, the impact of exercise, stress, and other factors.

The primary loop is calculating the blood glucose sensitivity as a function of carbs intake, insulin, exercise, and other factors, during different times of the day and different living conditions such as stress, menstrual period and environmental temperature. However, calculating these 12-36 sensitivity parameters needs a lot of data and stimuli of the body system to become accurate enough.

Therefore, the secondary learning loop that is based on big data from a community of other patients with T1D is introduced. Data that is collected from many patients are used to augment the learnings in the primary loop. There is no universal value to a specific sensitivity for a patient, but when lacking data to accurately calculate on an individual level, the values retrieved from the big data may be a good start that will augment the local patient-specific values.

In addition to supporting the primary loop, the big data analysis is used to understand how factors besides the regular carb/insulin/exercise will impact the BG balance of a T1D patient.

Brief description of the drawings

Figure 1 is a schematic overview illustration of the decision support system according to the present invention.
Figure 2 is a block diagram schematically illustrating the decision support system according to the present invention.
Figure 3 is a block diagram schematically illustrating an embodiment of the decision support system according to the present invention.
Figure 4 is a flow diagram schematically illustrating the method according to the present invention.

Detailed description

The decision support system and the method in relation to the system will now be described in detail with references to the appended figures. Throughout the figures, the same, or similar, items have the same reference signs.

Moreover, the items and the figures are not necessarily to scale, emphasis instead being placed upon illustrating the principles of the invention.

Given the development of CGM/FGM, SmartPhones, Artificial Intelligence and Big Data the inventor has realized intelligent ways of dealing with all day to day decisions taken by a T1D patient.

Some examples of the questions where a decision support system is needed are:

- I am going to eat 20 g of carbs, how much bolus do I need to stay within the BG range?
- I am low (BG<4 mmol/l), how many grams of carbs do I need to eat to get back to normal?
- My current BG is 10.2 mmol/l with is too high, how much bolus do I need to get back to normal?
- I am going to go running for 45 min, how much carbs do I need to stay within the range?
- Is my basal insulin titrated to a stable enough level?

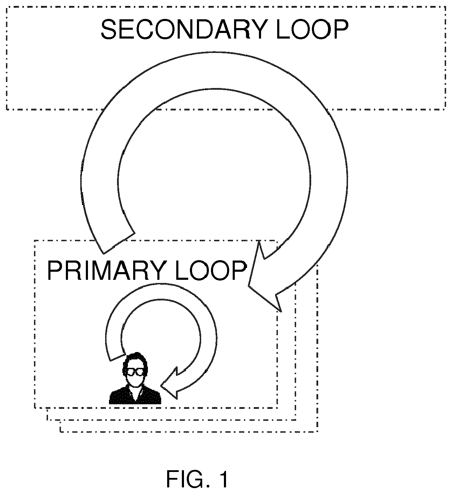

The decision support system according to the present invention is based upon a double learning loop system. The decision support system is schematically illustrated in figure 1.

The primary (personal) loop is using the data that is available from one patient to make the best possible estimate of a set of features. The secondary (cluster) loop is using data from many patients that are clustered to improve the personal feature estimation to improve the accuracy and thereby the decision accuracies. In parallel, the secondary loop is adding best-practice knowledge based on a set of defined and commonly known scenarios.

The primary and secondary loops comprise a number of blocks. Herein a block is a functional unit capable of receiving input signals representing physical entities, calculating various output parameters based upon the physical entities using predetermined formulas and algorithms. A block comprises one or many processing units, storage units, and communication units to communicate with other blocks of the system preferably using e.g. internet protocols or other communication protocols with appropriate security.

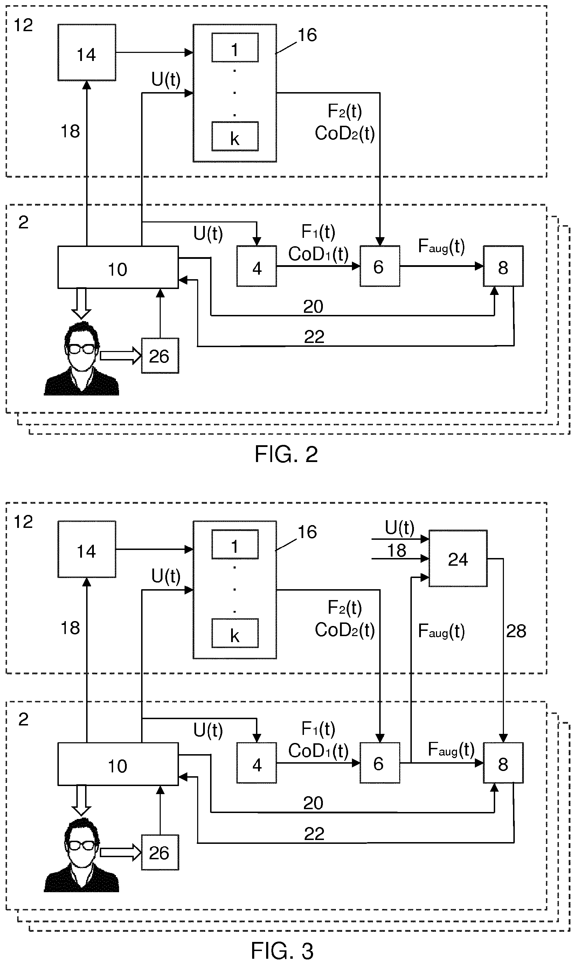

First with references to figures 1 and 2 a decision support system is provided, configured to support patients with Type 1 Diabetes, T1D, in their daily decision making, to determine a decision advice including timing and dosage of insulin.

The support system comprises a primary module 2 facilitating a primary loop related to each patient belonging to a community of several patients with T1D. The primary module 2 comprises a primary feature estimation block 4, a feature augmentation block 6, a decision advice block 8, and a control unit 10. This structure is the same for all patients. In figure 2 it is indicated two further primary modules of two other patients belonging to the community. In the figure the block-arrow from the patient indicates measured parameters, output instructions submitted by the patient, e.g. a decision request signal, and/or other parameters related to the patient, e.g. patient data, etc., and the block-arrow to the patient indicates information to the patient, e.g. a decision advice, and/or an information signal or a control signal to a medical unit.

The primary feature estimation block 4 is configured to receive a measurement vector U(t) related to the patient, and to determine a primary feature vector $F_1(t)$, and a primary coefficient of determination $CoD_1(t)$. $F_1(t)$ includes a set of feature vector elements, including at least insulin sensitivity, and carbohydrate sensitivity, and preferably also exercise sensitivity. $CoD_1(t)$ may be regarded as a measure of the accuracy of the estimated model in relation to data in the measurement vector U(t). CoD(t) is determined in accordance with the following formula:

$$CoD(t) = R^2 \left[ U(t),\, F(t) \right]$$

$R^2$ is a statistic that will give some information about the goodness of fit of a model. In regression, the $R^2$ coefficient of determination is a statistical measure of how well the regression predictions approximate the real data points. An $R^2$ of 1 indicates that the regression predictions perfectly fit the data.

The set of feature vector parameters preferably also includes estimated HbAlc (Glycosylated Hemoglobin, Type A1C), BG within range and area under the BG curve above limits.

The measurement vector U(t) from the patient comprises measurement parameters including at least blood glucose, injected insulin, consumed carbohydrates, and preferably also body temperature if above normal, pulse, and stress level. The primary module 2 comprises a measurement arrangement 26 that comprises one or many sensors configured to measure the measurement parameters of the measurement vector U(t). In the background section it is given numerous example of blood glucose sensors that may be applied.

The feature vector is a set of values derived from the measurement vector and is based on one patient only - this is performed by the primary feature estimation block.

However, an accurate estimation of the feature vector requires a substantial amount of observations. Therefore, the feature vector is augmented with data from a cluster of similar patients - this is performed by the feature augmentation block. Finally, this feature vector, together with specific advice derived from the analysis of a cluster of patients, is used to advise how to deal with a particular condition - this is performed by the decision advice block. Thus, the augmented feature vector is used to define a model of the patient that is used to evaluate the given hypothesis for treatment.

The decision support system further comprises a secondary module 12 facilitating a secondary loop being a learning loop configured to apply organized data from the community of several patients with T1D, and that said primary module 2 and said secondary module 12 are involved in the decision making for each patient belonging to the community. The secondary module 12 comprises a clustering of patients block 14, and a feature estimation per cluster block 16.

The clustering of patients block 14 is configured to receive patient data 18 from patients in the community, and to cluster the patients into k clusters of patients with similar patient data characteristics. k may be a variable number.

The clustering of patients block 14 is configured to perform a k-means clustering procedure, a k nearest neighbours clustering procedure, or similar clustering method/technique.

The following organized patient data may be used for the clustering:

T1D, weight, height, waist & hip, diabetes debut year, basal insulin type, bolus insulin type, physical activity work/pleasure per day, occupation activity habits, education level, family situation, menstruations, breastfeeding, pregnant, etc.

Further data may also be applied: Other glucose lowering drugs, other drugs, HbA1c, C-peptide, blood pressure, microalbumin, creatinine, retinopathy, neuropathy, cardiovascular disease, basal insulin injection site, bolus insulin injection site, tanner scale for puberty, etc.

It should be noted that for all patients of the community of patients, the same set of patient data parameters is applied.

The clustering of patients with similar characteristics is performed in a multi-dimensional space - performed by the clustering of patients block. Based on these clusters, the feature vector for each cluster is estimated based on the collection of all observations - this is performed by the feature estimation per cluster block. By using many patients for this, i.e., providing more observations, the accuracy is improved.

Thus, the patients are clustered with methods like K-means clustering. Preferably, the clustering aims to partition p observations into k clusters in which each observation belongs to the cluster with the nearest mean. This is an unsupervised learning method to identify clusters of patients with similar characteristics. K-means clustering is a method of vector quantization, originally from signal processing, that is popular for cluster analysis in data mining. K-means clustering aims to partition n observations into k clusters in which each observation belongs to the cluster with the nearest mean, serving as a prototype of the cluster. This results in a partitioning of the data space into Voronoi cells.

The feature estimation per cluster block 16 is configured to receive, for each cluster, measurement vectors U(t) of patients that belong to that cluster, and to calculate, for each of said k clusters, an estimated secondary feature vector, $F_2(t)$, and a secondary coefficient of determination, $CoD_2(t)$. $F_2(t)$ includes a set of estimated feature vector elements, including at least insulin sensitivity, and carbohydrate sensitivity, and $CoD_2(t)$ represents the accuracy of the estimated model.

The feature estimation per cluster block 16 is configured to transfer $F_2(t)$ and $CoD_2(t)$ to the feature augmentation blocks of patients that belong to the cluster of which the data has been estimated.

For each patient, the feature augmentation block 6 is configured to receive the $F_1(t)$ and $CoD_1(t)$ from the primary feature estimation block, and $F_2(t)$ and $CoD_2(t)$ from the feature estimation per cluster block 16 from the cluster to which the patient belongs, and to determine an augmented feature vector $F_{aug}(t)$, in dependence thereof.

The decision advice block 8 is configured to receive a decision request signal 20 from the control unit 10 and the augmented feature vector $F_{aug}(t)$, and to determine a decision advice 22, including timing and dosage of insulin, in dependence thereto.

The timing should be interpreted broadly and may relate to timing in relation to various events, e.g. insulin bolos intake, carbs intake and exercise. This will provide a tool to timely schedule these events to obtain predictable and stable blood glucose levels.

The decision request signal 20 may include a request from the patient for obtaining a decision advice in the present situation, or may include a decision advice hypothesis, e.g. "in the present situation I suggest 3 Units", that should be validated in the decision advice block 8. The hypothesis included in the decision request signal may thus cover the entire range from not having any hypothesis and only sending a question to the system regarding an advice, to a complete suggestion that the system may validate or adjust.

According to one embodiment the primary feature estimation block is configured to perform a regression based feature estimation, preferably a linear regression-based feature estimation, or a polynomial regression-based feature estimation.

The primary feature vector $F_1(t)$ and the secondary feature vector $F_2(t)$ include the same type of feature vector elements.

In one embodiment the augmented feature vector $F_{aug}(t)$ is a function on the vectors $F_1(t)$ and $F_2(t)$ balancing the estimates from said primary and secondary loop.

$$F_{aug}(t) = F_1 \, W_1 + F_2 \, W_2$$

$W_1$ and $W_2$ are weight matrices determined from $CoD_1$ and $CoD_2$.

$F_1$ is a vector comprising the elements $f_{1l}$ ... $f_{1m}$, and $F_2$ is a vector comprising the elements $f_{2l}$ ... $f_{2m}$. $CoD_1$ is a vector comprising the elements $r^2{}_{11}$ ...$r^2{}_{1m}$, and $CoD_2$ is a vector comprising the elements $r^2{}_{21}$ ... $r^2{}_{2m}$.

Where:

$$W_1 = \begin{bmatrix} w_{11} & \cdots & 0 \\ \vdots & \ddots & \vdots \\ 0 & \cdots & w_{1m} \end{bmatrix}$$

$$W_2 = \begin{bmatrix} w_{21} & \cdots & 0 \\ \vdots & \ddots & \vdots \\ 0 & \cdots & w_{2m} \end{bmatrix}$$

$$w_{ij} = \frac{r_{ij}^2}{r_{1j}^2 + r_{2j}^2}$$

If the dimension is only 1, and when the secondary loop, i.e. the cluster, gives four times as accurate data as the primary loop, this function can be as simple as:

$$F_{aug}(t) = 0{,}2 \cdot F_1 + 0{,}8\, F_2$$

In one further embodiment, illustrated in figure 3, the secondary module 12 also comprises a pattern recognition block 24 configured to receive measurement vectors from each cluster of patients, and patient data 18 and $F_{aug}(t)$ from a patient. In the pattern recognition block 24, patient data from the patient is matched with predefined type patterns, and at least one direct advice 28 is identified from the type patterns and transferred to the decision advice block 8 of the patient to further improve the decision advice provided to the individual patient. The pattern recognition block comprises a database of typical situations identified patient-by-patient based on clinical knowledge, and comprises all patient data of all patients as well as the individual measurement vectors, including the augmented feature vector $F_{aug}(t)$ calculated in the primary loop. For the remaining parts of the block diagram shown in figure 3, it is referred to the above description of figure 2.

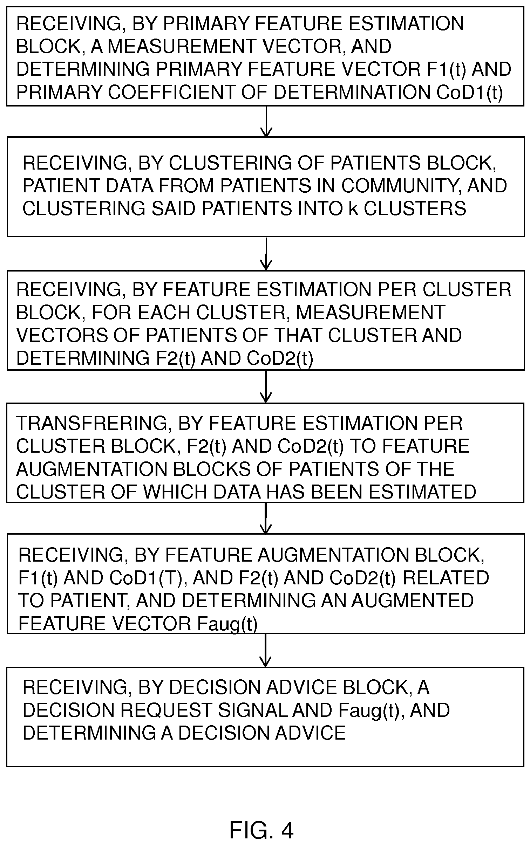

The present invention also relates to a method in relation to a decision support system. The method will be described with references to the flow diagram shown in figure 4. The decision support system has been described in detail above and it is herein referred to that description.

Thus, the decision support system is configured to support patients with Type 1 Diabetes, T1D, in their daily decision making, to determine a decision advice including timing and dosage of insulin. The support system comprises a primary module 2 facilitating a primary loop related to each patient belonging to a community of several patients with T1D. The primary module 2 comprises a primary feature estimation block 4, a feature augmentation block 6, a decision advice block 8, and a control unit 10.

The method comprises receiving, by the primary feature estimation block 6, a measurement vector U(t) related to the patient, and determining a primary feature vector $F_1(t)$, and a primary coefficient of determination $CoD_1(t)$. $F_1(t)$ includes a set of feature vector elements, including at least insulin sensitivity, and carbohydrate sensitivity, and $CoD_1(t)$ is a measure of the accuracy of an estimated model in relation to data in the measurement vector U(t).

The system further comprises a secondary module 12 facilitating a secondary loop being a learning loop configured to apply organized patient data from the community of several patients with T1D. The primary module 2 and the secondary module 12 are involved in the decision making for each patient belonging to the community. The secondary module 12 comprises a clustering of patients block 14, and a feature estimation per cluster block 16. The method further comprises:

- receiving, by said clustering of patients block 14, patient data 18 from patients in said community, and clustering said patients into k clusters of patients with similar patient data characteristics,
- receiving, by said feature estimation per cluster block 16, for each cluster, measurement vectors U(t) of patients that belong to that cluster, and calculating for each of said k clusters, an estimated secondary feature vector, $F_2(t)$,

and a secondary coefficient of determination, $CoD_2(t)$, wherein $F_2(t)$ includes a set of estimated feature vector elements, including at least insulin sensitivity, and carbohydrate sensitivity, and $CoD_2(t)$ represents an accuracy of an estimated model,

- transferring, by said feature estimation per cluster block 16, $F_2(t)$ and $CoD_2(t)$ to the feature augmentation blocks of patients that belong to the cluster of which the data has been estimated,
- receiving, by said feature augmentation block 6, said $F_1(t)$ and $CoD_1(t)$ from said primary feature estimation block, and $F_2(t)$ and $CoD_2(t)$ from said feature estimation per cluster block 16 from the cluster to which the patient belongs, and determining an augmented feature vector $F_{aug}(t)$, in dependence thereof, and

receiving, by said decision advice block 8, a decision request signal 20 from said control unit 10 and said augmented feature vector $F_{aug}(t)$, and determining a decision advice (22), including timing and dosage of insulin, in dependence thereto.

According to one embodiment the clustering comprises performing a K-means clustering procedure, or a k nearest neighbours clustering procedure, or other clustering method. The method preferably comprises performing a regression based feature estimation by the primary feature estimation block.

In a further embodiment the method comprises determining the augmented feature vector $F_{aug}(t)$. This has been described above in relation to the description of the decision support system, and it is referred to that description.

The method preferably comprises receiving, by a pattern recognition block provided in said secondary module 12, measurement vectors from each cluster of patients, and patient data and $F_{aug}(t)$ from a patient, and matching patient data from the patient with predefined type patterns, and identifying at least one direct advice from the type patterns and transferring said at least one direct advice to the decision advice block of said patient. Also this embodiment has been described in detail above and it is referred to that description.

The following table summarizes the information flow of the double loop concept:

| Information flow | Description | Information flow | Description |
|---|---|---|---|
| **Measurement vector** | The vector of time series of measurements from each patient | $F_1$ | The vector of features estimated from the specific patient only |
| **Patient data** | Vector of stationary or semi-stationary data from each patient | $CoD_1$ | The coefficient of determination for the primary loop estimation |
| **Hypothesis to be tested (decision request** signal) | The patient-initiated hypothesis for the decision to be verified | $F_2$ | The vector of features estimated in the secondary loop |
| **Decision advice** | Direct advice provided from the analysis of type patterns | $CoD_2$ | The coefficient of determination in the secondary loop |
| **Direct advice (optional)** | A set of advice identified based on a large set of patients and clinical knowledge | $F_{aug}$ | The vector of augmented features |
| **k-cluster** | Number of patient clusters identified | | |

The focus of the primary learning loop is to estimate a feature vector that can be used both to follow the medical status of the patient as well as a basis for decision support provided to the patient. For T1D the features may be, e.g., insulin and carb sensitivity but also other factors such as estimated HbA1c, BG within range and area under the BG curve above limits.

Other measurements such as stress and cortisol could be added. Measurement parameters used to estimate the feature vector comprises at least blood glucose, injected insulin, consumed carbohydrates, and preferably also pulse, stress level, and a cortisol value. Regression techniques are used to estimate the feature vector. Distinct events such as meals and exercise are needed to excite the system. A typical day may result in four to eight such events. As a consequence, many events and days are needed to get an estimate with high accuracy. To improve accuracy, the augmentation of the primary loop estimation by the secondary loop is needed.

The primary estimation is preferarably performed by a regression based feature estimation.

In the regression of the feature vector $F_1$, the coefficient of determination, $CoD_1$, is calculated. This is a measure of how well the estimated model fits with the observation data in the measurement vector. $CoD_1$ will be used to calculate the weight factor when augmenting the primary loop feature vector $F_1$ with the feature vector $F_2$ from a cluster of similar patients. The estimation is based on a sliding time window in order to keep the estimates updated with the latest state of the body system.

The second block of the primary module is the feature augmentation block. In this block, the augmented feature vector $F_{aug}$ is determined, which has been described above.

The third block of the primary loop, the decision advice block, is for testing personal hypothesis and providing the patient with a decision advice about how to control their T1D. The augmented feature vector $F_{aug}(t)$ is applied to the block.

According to one embodiment direct advice data 28 which is derived from patient data is received from the pattern recognition block in the secondary loop, and also a hypothesis, i.e. the decision request signal 20, to be tested, which is received from the primary loop. An estimated measurement vector $\widehat{Y(t)}$ based upon hypothesis of direct advice is then calculated.

$$\widehat{Y(t)} = \begin{pmatrix} \widehat{y1} \\ ... \\ ... \\ \widehat{yq} \end{pmatrix} = \begin{pmatrix} \widehat{BG} \\ \widehat{Carb} \\ \widehat{BolusIns} \\ \widehat{Exercise} \\ BasalIns \\ ... \\ ... \end{pmatrix}$$

Thus, the augmented feature vector is used to test the hypothesis that is provided by the patient. This could, e.g., be:

- What will happen if I take 2 U more of bolus insulin to compensate for a high BG value?
- I am going to go cross country skiing for the next hour, how much should I reduce my bolus insulin for lunch?

As an example, the estimated BG after two hours may be calculated as:

$$\widehat{BG} = BG_0 + PlannedBolus\ InsSense + PlannedCarbs\ CarbSens$$

Where *BG* is the estimated BG after two hours based on a hypothesis to inject and $BG_0$ is the current blood glucose level. *PlannedBolus* is the number of units of bolus insulin and eating *PlannedCarbs* is grams of carbs. *InsSense* and *CarbSens* are elements from the augmented feature vector.

Thus, this block is configured to generate an estimated measurement vector with the estimated measurements based on the hypothesis and/or other advice. It is always up to the individual patient to judge if the proposed decision should be followed. The system can never take this responsibility, how accurate the estimations are.

The secondary learning loop is driven by (anonymized) data from each patient in the community using the system. Since big data technology is available the amount of data is not a problem and 15 minute BG samples together with event data such as carb intake, exercise and stress can be exported to the cloud of shared data. Each patient represents an object in the data and is also described with information that is necessary for a relevant clustering of the patients. These data are, e.g., age, weight, length, gender, activity level, regularity of life, time with T1D, other diseases, etc.

The patient data, PD, for complete population of p individuals is input to the K-means clustering block, where p is the number of patients, and q is the number of data points per patient.

$$PD = \begin{matrix} p_{11} & \cdots & p_{1q} \\ p_{21} & \cdots & p_{2q} \\ p_{31} & \cdots & p_{3q} \\ & \cdots & \\ p_{p1} & \cdots & p_{pq} \end{matrix}$$

The second block performs one feature estimation per cluster. The estimation of sensitivity factors concerning insulin, carbs, exercise, and stress are then carried out on much more data than on patient level but using the same type of regression method as in the primary loop. The estimation error as the Coefficient of Determination CoD2 is calculated for each regression. The Feature vector F2, as well as the CoD2, is transferred to the patients that belong to the cluster of which the data has been estimated. This may be performed continuously, or each time a new feature augmentation should be performed in a primary loop of a patient.

The final block of the secondary module is the pattern recognition block which is an optional block. This block has as input all data of all patients as well as the individual measurement vectors, including the augmented feature vector $F_{aug}(t)$ calculated in the primary loop. The type patterns are common situations that have been identified by clinical specialists in advance. In the pattern recognition block, individual patient data is matched with the type patterns. The direct advice is identified from one or several type patterns and transferred to the patient-specific primary loop module.

The following listing give some examples of direct advice that could be derived from patient data. The direct advice is generated from patterns that are diagnosed by clinical specialists on a generic level. Example of patterns to be identified:

- Possible to improve BG level by adjusting the dosing of the basal insulin or possible change insulin type (consult your HCP).
- Possible to improve BG peaks after meals by an earlier administration of bolus insulin (approx. 30 min before meal).
- Make sure not to overcompensate for small hypoglycemia episodes by eating too much carbs.
- Be aware of that intensive physical activities may have an impact on your BG also 12 hour after the activity.
- Working days and days off are very different. Consider having a more regular and even profiles for all days of a week.
- Your BG profiles seems to be impacted by your menstruation cycle. Please consult your HCP to find a way to proactively deal with this pattern.

The advice may be structured in a set of positive feedback issues "doing good" and a number of improvement proposals. The set of feedback is automatically identified and prioritized by the pattern recognition in the pattern recognition block 24.

Below is an example illustrating the use of the decision support system:
Assume the patient has a Blood Glucose of 4.1 mmol/l. He is going to have lunch and go skiing for 10 km after lunch. The lunch is a nice pasta with 80 g of carbohydrates. 10 km of skiing is approx. 400 kcal in burned energy.

He enters, as a decision request signal, the BG, the 80 g of carb and the 400 kcal. He also makes a hypothesis that this will not require more than 4 U of bolus insulin. The system checks and delivers an answer that this will bring the BG to dangerously low levels of 2 mmol after two hours. Therefore he will test with 2 U instead and this gives a BG of 6 mmol/l which is on the right side. He decides to follow this advice and confirms that he injects this amount of bolus insulin.

The present invention is not limited to the above-described preferred embodiments. Various alternatives, modifications and equivalents may be used. Therefore, the above embodiments should not be taken as limiting the scope of the invention, which is defined by the appending claims.

**Claims**

1. A decision support system configured to support patients with Type 1 Diabetes, TID, in their daily decision making, to determine a decision advice including timing and dosage of insulin, the support system comprises a primary module (2) facilitating a primary loop related to each patient belonging to a community of several patients with T1D, the primary module (2) comprises a primary feature estimation block (4), a feature augmentation block (6), a decision advice block (8), and a control unit (10), said primary feature estimation block (6) is configured to receive a measurement vector U(t) related to said patient, and to determine a primary feature vector $F_1(t)$, and a primary coefficient of determination $CoD_1(t)$, wherein said $F_1(t)$ includes a set of feature vector elements, including at least insulin sensitivity, and carbohydrate sensitivity, and $CoD_1(t)$ is a measure of the accuracy between an estimated model in relation to data in the measurement vector $U(t)$, **characterized in that** said system further comprises a secondary module (12) facilitating a secondary loop being a learning loop configured to apply organized data from said community of several patients with T1D, and that said primary module (2) and said secondary module (12) are involved in the decision making for each patient belonging to said community, wherein said secondary module (12) comprises a clustering of patients block (14), and a feature estimation per cluster block (16),

said clustering of patients block (14) is configured to receive patient data (18) from patients in said community, and to cluster said patients into k clusters of patients with similar patient data characteristics,

said feature estimation per cluster block (16) is configured to receive, for each cluster, measurement vectors $U(t)$ of patients that belong to that cluster, and to calculate, for each of said k clusters, an estimated secondary feature vector, $F_2(t)$, and a secondary coefficient of determination, $CoD_2(t)$, wherein $F_2(t)$ includes a set of estimated feature vector elements, including at least insulin sensitivity, and carbohydrate sensitivity, and $CoD_2(t)$ represents an estimated error, and wherein said feature estimation per cluster block (16) is configured to transfer $F_2(t)$ and $CoD_2(t)$ to the feature augmentation blocks of patients that belong to the cluster of which the data has been estimated,

said feature augmentation block (6) is configured to receive said $F_1(t)$ and $CoD_1(t)$ from said primary feature estimation block, and $F_2(t)$ and $CoD_2(t)$ from said feature estimation per cluster block (16) from the cluster to which the patient belongs, and to determine an augmented feature vector $F_{aug}(t)$, in dependence thereof, and

said decision advice block (8) is configured to receive a decision request signal (20) from said control unit (10) and said augmented feature vector $F_{aug}(t)$, and to determine a decision advice (22), including timing and dosage of insulin, in dependence thereto.

2. The decision support system according to claim 1, wherein the primary feature estimation block is configured to perform a regression-based feature estimation.

3. The decision support system according to claim 1 or 2, wherein said patient data comprises one or many of age, weight, length, gender, activity level, regularity of life, time with T1D, other diseases, etc., for each patient.

4. The decision support system according to any of claims 1-3, wherein $F_1(t)$ and $F_2(t)$ include the same type of feature vector elements.

5. The decision support system according to any of claims 1-4, wherein said augmented feature vector $F_{aug}(t)$ is a function on the vectors $F_1(t)$ and $F_2(t)$ balancing the estimates from said primary and secondary loop.

6. The decision support system according to any of claims 1-5, wherein said clustering block is configured to perform a K-means clustering procedure, or a k nearest neighbors clustering procedure.

7. The decision support system according to any of claims 1-6, wherein said secondary module (12) also comprises a pattern recognition block configured to receive measurement vectors from each patient, and patient data and $F_{aug}(t)$ from a patient, wherein in said pattern recognition block, patient data from said patient is matched with predefined type patterns, and at least one direct advice is identified from said type patterns and transferred to the decision advice block of said patient.

8. The decision support system according to claim 7, wherein said pattern recognition block comprises a database of typical situations identified patient-by-patient based on clinical knowledge, and comprises all patient data of all patients as well as the individual measurement vectors, including the augmented feature vector $F_{aug}(t)$ calculated in the primary loop.

9. The decision support system according to any of claims 1-8, wherein said primary module (2) comprises a measurement arrangement (26), and wherein said measurement arrangement comprises one or many sensors configured to measure parameters of said measurement vector $U(t)$.

10. The decision support system according to any of claims 1-9, wherein said measurement vector $U(t)$ from the patient comprises measurement parameters including at least blood glucose, injected insulin, consumed carbohydrates, body temperature if above normal, and pulse, and preferably also stress level.

11. A method in relation to a decision support system configured to support patients with Type 1 Diabetes,TID, in their daily decision making, to determine a decision advice including timing and dosage of insulin, the support system comprises a primary module (2) facilitating a primary loop related to each patient belonging to a community of several patients with T1D, the primary module (2) comprises a primary feature estimation block (4), a feature augmentation block (6), a decision advice block (8), and a control unit (10), the method comprises:

- receiving, by said primary feature estimation block (6), a measurement vector U(t) related to said patient, and determining a primary feature vector $F_1(t)$, and a primary coefficient of determination $CoD_1(t)$, wherein said $F_1(t)$ includes a set of feature vector elements, including at least insulin sensitivity, and carbohydrate sensitivity, and $CoD_1(t)$ is a measure of the accuracy between an estimated model in relation to data in the measurement vector U(t), **characterized in that** said system further comprises a secondary module (12) facilitating a secondary loop being a learning loop configured to apply organized data from said community of several patients with T1D, and that said primary module (2) and said secondary module (12) are involved in the decision making for each patient belonging to said community, wherein said secondary module (12) comprises a clustering of patients block (14), and a feature estimation per cluster block (16), said method further comprises:

- receiving, by said clustering of patients block (14), patient data (18) from patients in said community, and clustering said patients into k clusters of patients with similar patient data characteristics,

- receiving, by said feature estimation per cluster block (16), for each cluster, measurement vectors U(t) of patients that belong to that cluster, and calculating for each of said k clusters, an estimated secondary feature vector, $F_2(t)$, and a secondary coefficient of determination, $CoD_2(t)$, wherein $F_2(t)$ includes a set of estimated feature vector elements, including at least insulin sensitivity, and carbohydrate sensitivity, and $CoD_2(t)$ represents a measure of the accuracy of an estimated model,

- transferring, by said feature estimation per cluster block (16), $F_2(t)$ and $CoD_2(t)$ to the feature augmentation blocks of patients that belong to the cluster of which the data has been estimated,

- receiving, by said feature augmentation block (6), said $F_1(t)$ and $CoD_1(t)$ from said primary feature estimation block, and $F_2(t)$ and $CoD_2(t)$ from said feature estimation per cluster block (16) from the cluster to which the patient belongs, and determining an augmented feature vector $F_{aug}(t)$, in dependence thereof, and

receiving, by said decision advice block (8), a decision request signal (20) from said control unit (10) and said augmented feature vector $F_{aug}(t)$, and determining a decision advice (22) in dependence thereto.

12. The method according to claim 11, comprising performing a regression based feature estimation by said primary feature estimation block.

13. The method according to claim 11 or 12, wherein said augmented feature vector $F_{aug}(t)$ is a function on the vectors $F_1(t)$ and $F_2(t)$ balancing the estimates from said primary and secondary loop.

14. The method according to any of claims 11-13, wherein said clustering comprises performing a K-means clustering procedure, or a k nearest neighbours clustering procedure.

15. The method according to any of claims 11-14, comprising receiving, by a pattern recognition block provided in said secondary module (12), measurement vectors from each patient, and patient data and $F_{aug}(t)$ from a patient, and matching patient data from said patient with predefined type patterns, and identifying at least one direct advice from said type patterns and transferring said at least one direct advice to the decision advice block of said patient.

FIG. 1

FIG. 2

FIG. 3

RECEIVING, BY PRIMARY FEATURE ESTIMATION BLOCK, A MEASUREMENT VECTOR, AND DETERMINING PRIMARY FEATURE VECTOR F1(t) AND PRIMARY COEFFICIENT OF DETERMINATION CoD1(t)

↓

RECEIVING, BY CLUSTERING OF PATIENTS BLOCK, PATIENT DATA FROM PATIENTS IN COMMUNITY, AND CLUSTERING SAID PATIENTS INTO k CLUSTERS

↓

RECEIVING, BY FEATURE ESTIMATION PER CLUSTER BLOCK, FOR EACH CLUSTER, MEASUREMENT VECTORS OF PATIENTS OF THAT CLUSTER AND DETERMINING F2(t) AND CoD2(t)

↓

TRANSFRERING, BY FEATURE ESTIMATION PER CLUSTER BLOCK, F2(t) AND CoD2(t) TO FEATURE AUGMENTATION BLOCKS OF PATIENTS OF THE CLUSTER OF WHICH DATA HAS BEEN ESTIMATED

↓

RECEIVING, BY FEATURE AUGMENTATION BLOCK, F1(t) AND CoD1(T), AND F2(t) AND CoD2(t) RELATED TO PATIENT, AND DETERMINING AN AUGMENTED FEATURE VECTOR Faug(t)

↓

RECEIVING, BY DECISION ADVICE BLOCK, A DECISION REQUEST SIGNAL AND Faug(t), AND DETERMINING A DECISION ADVICE

FIG. 4

Europäisches
Patentamt

European
Patent Office

Office européen
des brevets

## EUROPEAN SEARCH REPORT

Application Number

EP 19 17 0910

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/053101 A1 (BOOTH ROBERT C [US] ET AL) 23 February 2017 (2017-02-23)<br>* abstract *<br>* paragraphs [0005] - [0011], [0034] - [0036], [0048], [0049], [0055], [0056], [0067] - [0071], [0080], [0087] - [0090], [0094] * | 1-15 | INV.<br>G16H20/17<br>G16H50/20 |
| | ----- | | |
| X | US 2016/354543 A1 (CINAR ALI [US] ET AL) 8 December 2016 (2016-12-08)<br>* abstract *<br>* paragraphs [0009] - [0014], [0039], [0040], [0054] - [0089]; claims 1-5 * | 1-15 | |
| | ----- | | |
| Y | ELENI I GEORGA ET AL: "Multivariate Prediction of Subcutaneous Glucose Concentration in Type 1 Diabetes Patients Based on Support Vector Regression", IEEE JOURNAL OF BIOMEDICAL AND HEALTH INFORMATICS, IEEE, PISCATAWAY, NJ, USA, vol. 17, no. 1,<br>1 January 2013 (2013-01-01), pages 71-81, XP011515041,<br>ISSN: 2168-2194, DOI: 10.1109/TITB.2012.2219876<br>* the whole document * | 1-15 | TECHNICAL FIELDS SEARCHED (IPC)<br><br>G16H |
| | ----- | | |
| | -/-- | | |

The present search report has been drawn up for all claims

| Place of search | Date of completion of the search | Examiner |
|---|---|---|
| Munich | 18 October 2019 | Thumb, Werner |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

Application Number

EP 19 17 0910

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | Klaus Donsa ET AL: "Towards Personalization of Diabetes Therapy Using Computerized Decision Support and Machine Learning: Some Open Problems and Challenges" In: "International Conference on Computer Analysis of Images and Patterns. CAIP 2017: Computer Analysis of Images and Patterns", 1 January 2015 (2015-01-01), Springer, Berlin, Heidelberg 032548, XP55381519, ISBN: 978-3-642-17318-9 vol. 8700, pages 237-260, DOI: 10.1007/978-3-319-16226-3_10, * the whole document * * in particular sections 4.1 and 5.2 * ----- | 1-15 | |
| A | MANISH GUTCH ET AL: "Assessment of insulin sensitivity/resistance", INDIAN JOURNAL OF ENDOCRINOLOGY AND METABOLISM, vol. 19, no. 1, 1 January 2015 (2015-01-01), page 160, XP55633410, ISSN: 2230-8210, DOI: 10.4103/2230-8210.146874 * the whole document * ----- | 1-15 | TECHNICAL FIELDS SEARCHED (IPC) |

The present search report has been drawn up for all claims

| Place of search | Date of completion of the search | Examiner |
|---|---|---|
| Munich | 18 October 2019 | Thumb, Werner |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 17 0910

This annex lists the patent family members relating to the patent documents cited in the above-mentioned European search report.
The members are as contained in the European Patent Office EDP file on
The European Patent Office is in no way liable for these particulars which are merely given for the purpose of information.

18-10-2019

| Patent document cited in search report | | Publication date | Patent family member(s) | | Publication date |
|---|---|---|---|---|---|
| US 2017053101 | A1 | 23-02-2017 | AU | 2016308953 A1 | 15-02-2018 |
| | | | CA | 2993275 A1 | 23-02-2017 |
| | | | EP | 3337402 A1 | 27-06-2018 |
| | | | JP | 2018523546 A | 23-08-2018 |
| | | | US | 2017053101 A1 | 23-02-2017 |
| | | | US | 2018122503 A1 | 03-05-2018 |
| | | | WO | 2017031440 A1 | 23-02-2017 |
| US 2016354543 | A1 | 08-12-2016 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20130345663 A
- US 20160070871 A
- US 20120123234 A
- US 20050278073 A
- WO 201811766 A
- US 2017216518 A